# EUROPEAN PATENT SPECIFICATION

(11) **EP 0 654 028 B1**
(45) Date of publication and mention of the grant of the patent: **17.04.1996**
(21) Application number: 93914656.9
(22) Date of filing: 08.07.1993
(51) Int. Cl.: C07D 273/00, C07D 413/12, A61K 31/41

(54) **3- AND 5-SUBSTITUTED 1,2,3,4-OXATRIAZOLE-5-IMINE COMPOUNDS, A PROCESS FOR THE PREPARATION THEREOF, A PHARMACEUTICAL PREPARATION CONTAINING SAID COMPOUNDS AND THE USE OF SAID COMPOUNDS FOR THE PREPARATION OF MEDICAMENTS**
3-UND 5-SUBSTITUIERTE 1,2,3,4,-OXATRIAZOL-5-IMIN DERIVATE, VERFAHREN ZU DEREN HERSTELLUNG, DIESE ENTHALTENDE ARZNEIMITTEL UND VERWENDUNG DIESER VERBINDUNGEN ZUR HERSTELLUNG VON ARZNEIMITTELN
COMPOSES DE 1,2,3,4-OXATRIAZOLE-5-IMINE SUBSTITUES EN POSITION 3 ET 5, LEUR PROCEDE DE PREPARATION, PREPARATION PHARMACEUTIQUE LES CONTENANT, ET LEUR APPLICATION A LA PREPARATION DE MEDICAMENTS

(30) Priority: 10.08.1992 DK 1003/92
(43) Date of publication of application: 24.05.1995
(73) Proprietor: A/S GEA Farmaceutisk Fabrik, DK-2000 Frederiksberg (DK)
(72) Inventor: KARUP, Gunnar, Leo, DK-2300 Copenhagen S (DK); PREIKSCHAT, Herbert, Fritz, DK-3460 Birkerod (DK); PEDERSEN, Soren, Bols, DK-2650 Hvidovre (DK); CORELL, Tim, Niss, DK-2800 Lyngby (DK); ALHEDE, Borge, Ingvar, Frisch, DK-2670 Greve Strand (DK)
(74) Representative: Mathiesen, Hans Preben
(86) International application number: DK9300234
(87) International publication number: WO9403442

(56) References cited:
- US-A- 4 329 355
- Chemical Abstracts, volume 73, no. 17, 26 October 1970, (Columbus, Ohio, US), see page 361, abstract 87922f, & JP,A,7021102 (N-acyl-anhydro-1,2,3,4-oxatriazole-5-imines
- Chemical Abstracts, volume 73, no. 17, 26 October 1970, (Columbus, OHIO, US), see page 362, abstract 87930g, & JP,A,7020904 (Mesoionic coumpounds)
- Acta chemica Scandinavia, Vol. 25, 1971, Carsten Christophersen et al: " The reaction of 1-substituted and 1,4-disubstituted thio- semicarbazides with Nitrous Acid. 3-substituted N-/5-(1,2,3,4-oxatriazolo)/amides"
- Chem. Phrm. Bull., Vol. 19, no. 3, 1971, Katsutada Masuda et al:"Studies on Mesoionic Compounds. III. Synthesis of -3-Acryl-5-imino-3,5-dihydro-1-oxa-2,3,4-triazole Hydrochlorides and their Derivatives"

## Description

### Technical Field

The present invention relates to hitherto unknown 3- and 5-substituted 1,2,3,4-oxatriazole-5-imine compounds having biological effects making them suitable for treatment of cardiovascular diseases (blood clots) and asthma, a process for the preparation thereof and a pharmaceutical preparation containing said compounds. Furthermore, the invention relates to the use of said compounds for the preparation of medicaments.

### Background Art

N.G. Finnegan et al., J. Org. Chem. 30, pages 567-575 (1965) discloses the compound 3-cyclohexyl-1,2,3,4-oxatriazole-5-imino-hydrochloride. However, no biological effect of said compound is mentioned.

K. Masuda et al., Chem. Pharm. Bull. 19 (3) pages 559-563 (1971) discloses 3-aryl-1,2,3,4-oxatriazole-5-imine compounds and acyl derivatives thereof, wherein the aryl group may be monosubstituted by methyl or halogen. Even though these compounds were synthesized in the hope of finding new hypotensive agents, no biological effects of the compounds are described.

C. Christophersen et al., Acta Chemica Scandinavira, 25, pages 625-630 (1971), discloses 3-substituted 1,2,3,4-oxatriazole-5-imino compounds, wherein the 3-substituent may be propyl or phenyl or cyclohexyl. However, no biological effects of said compounds are described.

Hanley et al., J.C.S. Perkin Trans I, 736-740 (1979), discloses 3-aryl-1,2,3,4-oxatrizole-5-imine compounds, wherein the aryl group may be monosubstituted by methyl or halogen. However, no biological effects of the compounds are described.

The JP Patents Nos. 20904/70 and 21102/70 disclose 3-substituted 1,2,3,4-oxatriazole-5-imine salts and acyl derivatives thereof, wherein the 3-substituent may be aryl, optionally monosubstituted by chlorine or methyl. The vasodepressor activity of said compounds is stated as a biological effect.

GB patent specification No. 2 015 878 discloses 3-phenyl-1,2,3,4-oxatriazole-5-imine compounds, for which a pesticidal and/or pest ovicidal and/or hebicidal activity has been found.

US patent specification No. 4,329,355 discloses anhydro-5-imino-1,2,3,4-oxatriazolium hydroxides of a structure similar to the structure of the compounds of the present invention. However, the compounds known from this patent specification are only mentioned as being useful in the treatment of cancer.

Furthermore, from J.C.S. Perkin Trans I, 747-751 (1979) compounds of a structure similar to the structure of the compounds of the present invention are known. However, no biological effects of said compounds have been stated.

### Disclosure of the Invention

The present invention relates to hitherto unknown 3- and 5-substituted 1,2,3,4,-oxatriazole-5-imine compounds of the general formula I 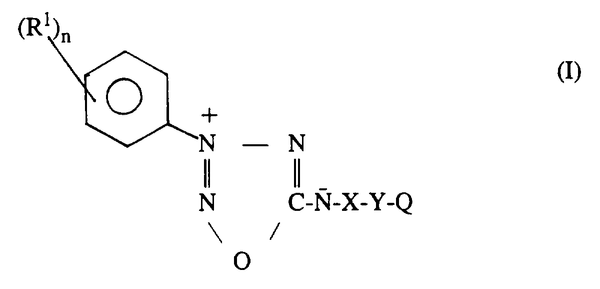 being characterised in that R¹ is the same or different groups and represents alkyl or alkoxy groups having 1 to 3 carbon atoms, halogen, trifluoromethyl, nitro, cyano, phenyl or alkylsulphonyl groups, n is 1 to 3, whereby R¹ is not halogen or alkyl, when n = 1,
X is -SO₂ or - C(O)NH-,
Y is -(CHR)ₘ-, wherein m = 1 to 4, and R means -CH₂-aryl, alkyl, hydrogen or a direct bond, and
Q means 10-camphoryl, -C(O)O-alkyl, aryl, -SO₂-alky- or -SO₂-aryl, where aryl means phenyl or 4-alkyl-1,3-thiazole-5-yl and the aryl group is substituted by 1 to 3 groups Z, where Z means -NH-C(O)-C₁₋₆alkyl, -C(O)O-C₁₋₆alkyl or -O-(CHR³)ₚ-OH, wherein p = 1 to 4 and R³ means H or OH, where Z may further mean methoxy, when the aryl group in -SO₂-aryl is a phenyl group.

The compounds according to the invention differ from the above prior art compounds by their chemical constitution, as they have a different substitution in the 3-position and/or in the 5-position of the oxatriazole ring, and they differ from the compounds known from the above patents with respect to their biological effect, as they inhibit the blood platelet aggregation and have a relaxation effect on the trachea.

The invention further relates to a pharmaceutical preparation being characterised in that it comprises as an active ingredient a compound of formula I together with a pharmaceutically acceptable carrier or diluent.

Moreover, the invention relates to a process for the preparation of said 3- and 5- substituted 1,2,3,4-oxatriazole-5-imine compounds of the general formula I, said process being characterised by ring closing a 1-arylthiosemicarbazide derivative of the general formula II 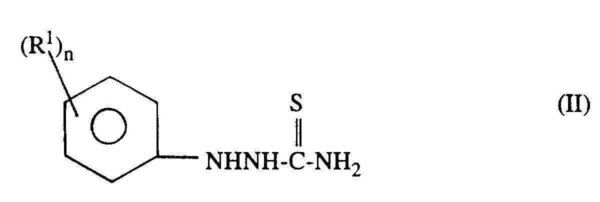 wherein R¹ and n have the same meaning as in formula I, by treatment with alkyl nitrite having 1 to 6 carbon atoms or alkali metal nitrite under acidic conditions at 0 to 10°C, whereafter the resulting salt is converted into the corresponding free compound, which is subsequently reacted with a compound of the type ClSO₂-Y-Q or O=C=N-Y-Q, wherein Y and Q have the same meaning as in formula I.

The ring closure reaction by the use of alkyl nitrite having 1 to 6 carbon is hitherto unknown and is preferred for the preparation of the compounds according to the invention, as a quantitative yield prior to purification is obtained hereby.

In the process according to the invention it is preferred to use ethyl nitrite as alkyl nitrite having 1 to 6 carbon atoms, and sodium nitrite is preferred as an alkali metal nitrite.

It is known per se to cyclize 1,4-disubstituted thiosemicarbazides with nitrous acid (sodium nitrite and acid) to form 3-substituted 1,2,3,4-oxatriazole-5-imines. The yields at this reaction are stated to be between 18 and 57%.

For reacting 1 equivalent of the 1-aryl-thiosemicarbazide derivative with alkyl nitrite having 1 to 6 carbon atoms, it is preferred to use 2 to 2.5 equivalents of alkyl nitrite in a suitable solvent, such as alkyl alcohol having 1 to 6 carbon atoms, to obtain a 3-arylsubstituted 1,2,3,4-oxatriazole-5-imine salt in a substantially quantitative yield. After filtration of the precipitated sulphur and evaporation of the solvent, the product is, if necessary, recrystallized from for instance alkyl alcohol having 1 to 6 carbon atoms, acetonitrile or nitromethane, whereby the yields of the pure product obtained are usually between 60 and 95%.

As alkyl alcohol having 1 to 6 carbon atoms methanol or ethanol is preferred.

The necessary starting compounds of the general formula II may be prepared in a manner known per se by reacting the corresponding arylhydrazine hydrochloride with an alkali thiocyanate or an ammonium thiocyanate in a suitable solvent, such as alcohol or water, using reflux for 6 to 18 hours, for instance as described by Houben-Weyl: "Methoden Der Organischen Chemie E4", page 513.

### PREPARATION OF THE STARTING MATERIALS

### Preparation of 1-(3-chloro-2-methylphenyl)thiosemicarbazide

19.3 g (0.1 mole) of 3-chloro-2-methylphenylhydrazine-hydrochloride were dissolved in 200 ml of absolute ethanol. 11.64 g (0.12 mole) of potassium thiocyanate were added to the solution, and the mixture was heated during reflux for 16 hours. The mixture was then cooled, whereby the product was partially precipitated, and the mixture was subsequently evaporated to dryness on a rotatory evaporator. The product was recrystallized from 200 ml water and 250 ml methanol, separated by filtration and washed thoroughly with water.
Yield: 17.8 g = 82.5%
Melting point: 192-193°C.

| Elemental analysis: C₈H₁₀ClN₃S: | | | | |
|---|---|---|---|---|
| Calculated | C: 44.54% | H: 4.67% | N: 19.48% | S:14.86% |
| Found | C: 44.22% | H: 4.58% | N: 19.60% | S:14.67% |

500 MHz ¹H NMR (d₆-DMSO):
δ 9.33 (br s, 1H, NH), δ 7.80 (br s, 1H, NH), δ 7.72 (br s, 1H, NH), δ 7.52 (br s, 1H, NH), δ 6.80 (m, 3H, ArH), δ 2.18 (s, 3H, CH₃).

### Preparation of 3-(3-chloro-2-methylphenyl)-1,2,3,4-oxatriazole-5-imine hydrochloride

8.6 g (40 mmole) of 1-(3-chloro-2-methylphenyl)thiosemicarbazide were dissolved in 100 ml of methanol and 5 ml of 37% hydrochloric acid while being stirred at room temperature. The mixture was cooled to 0 to 5°C by means of an ice bath, and 6.3 g (7 ml) of ethyl nitrite were subsequently added in small quantities over a period of approximately 5 minutes. The mixture became dark coloured by the nitrous vapours, but turned light after a few minutes at the same time as free sulphur precipitated. The mixture was stirred for 10 minutes, and additional 0.9 g (1 ml) of ethyl nitrite was then added, and the reaction mixture was then left for about 20 minutes while being stirred. The sulphur was separated by filtration and the mixture was evaporated on a rotating evaporator at a bath temperature of 30°C. If necessary, the mixture was dehydrated by evaporation together with toluene/ethanol. The crystals were stirred with diethyl ether, separated by filtration and washed further with small amounts of diethyl ether.
Yield: 9.2 g = 94%
Melting point: 194-195°C (decomposes)
IR: 1700 cm⁻¹.

| Elemental analysis: C₈H₇ClN₄O, HCl, ¼H₂O: | | | | |
|---|---|---|---|---|
| Calculated | C: 38.19% | H: 3.41% | N: 22.28% | Cl:28.18% |
| Found | C: 38.07% | H: 3.19% | N: 22.30% | Cl:28.58% |

500 MHz ¹H NMR (D₂O):
δ 7.52 (m, 3H, ArH), δ 2.38 (s, 3H, CH₃)

### Example 1

### 3-(3-chloro-2-methylphenyl)-1,2,3,4-oxatriazole-5-(N-phenyl sulphonyl carbamoyl) imine

4.94 g (20 mml) of 3-(3-chloro-2-methylphenyl)-1,2,3,4-oxatriazole-5-iminehydrocloride were dissolved in 100 ml of water and 2.1 g (25 mmole) of sodium hydrogencarbonate were subsequently added while being stirred. After terminated development of carbon dioxide 100 ml of chloroform were added, whereafter the precipitated substance dissolved. Under vigorous stirring 3.85 g (21 mmole) of benzene sulphonyl isocyanate were added to the mixture and stirring was continued for 60 minutes, whereby a precipitate was formed. This precipitate was separated by filtration and the chloroform phase was washed thrice with 50 ml of 1N hydrochloric acid and subsequently with water, whereafter it was concentrated. The product formed by the concentration was mixed with the precipitate separated by filtration and this mixture was subsequently stirred with a small quantity of ether, whereafter the mixture was separated by filtration and dried.
Yield: 6.7 g = 84.6%
Melting point: 163-165°C
IR: 1695 cm⁻¹, 1685 cm⁻¹, 1330 cm⁻¹, 1160 cm⁻¹ (N⁻-SO₂-); 1650 cm⁻¹ (N⁻-CO-NH).

| Elemental analysis: C₁₅H₂₂ClN₅SO₄: | | | | |
|---|---|---|---|---|
| Calculated | C: 45.46% | H: 3.07% | N: 17.79% | S:8.14% |
| Found | C: 45.46% | H: 3.14% | N: 17.31% | S:7.92% |

### Example 2

### 3-(3-chloro-2-methylphenyl)-1,2,3,4-oxatriazole-5-(N-2-acetamido-4-methyl-5-thiazole sulphamoyl)imine

1.95 g (7.9 mmole) of 3-(3-chloro-2-methylphenyl)-1,2,3,4-oxatriazole-5-imine hydrochloride were dissolved/suspended in 30 ml of pyridine, and 2.0 g (7.9 mmole) of 2-acetamido-4-methyl-5-thiazole sulphonyl chloride were subsequently added while being stirred. The mixture was stirred for 75 minutes at room temperature, whereafter it was poured into 350 ml of water while being stirred vigorously. The precipitated product was separated by filtration, washed thoroughly with water and diethyl ether and dried under vacuum.
Yield: 1.71 g = 50.4%
Melting point: 158-159°C
IR: 1690 cm⁻¹ (-NH-CO-) ; 1610 cm⁻¹ , 1320 cm⁻¹ , 1155 cm⁻¹ (N⁻-SO₂-)

| Elemental analysis: C₁₄H₁₂ClN₆O₄S₂,H₂O: | | | | |
|---|---|---|---|---|
| Calculated | C: 37.61% | H: 3.38% | N: 18.81% | S:14.34% |
| Found | C: 37.63% | H: 3.32% | N: 18.84% | S:14.60% |

### Example 3

### 3-(3-chloro-2-methylphenyl)-1,2,3,4-oxatriazole-5-(N-4-acetylamino phenyl sulphamoyl)imine

4.94 g (20 mmole) of 3-(3-chloro-2-methylphenyl)-1,2,3,4-oxatriazole-5-imine hydrochloride were dissolved/suspended in 70 ml of pyridine, and 4.67 g (20 mmole) of N-acetyl sulphanyl chloride were subsequently added, while being stirred. The mixture was then stirred for 75 minutes at room temperature and subsequently poured into 900 ml of water while being stirred vigorously. The precipitated product was separated by filtration, then washed thoroughly with water and diethyl ether and dried under vacuum.
Yield: 6.87 g = 84.2%
Melting point: 224-225°C
IR: 1700 cm⁻¹ (-NH-CO-); 1630 cm⁻¹, 1320 cm⁻¹, 1155 cm⁻¹ (N⁻-SO₂-)

| Elemental analysis: C₁₆H₁₄ClN₅SO₄: | | | | |
|---|---|---|---|---|
| Calculated | C: 47.12% | H: 3.46% | N: 17.18% | S: 7.86% |
| Found | C: 47.10% | H: 3.28% | N: 16.83% | S: 7.98% |

### Example 4

### 3-(3-chloro-2-methylphenyl)-1,2,3,4-oxatriazole-5-(N-(1S)-(+)-10-camphoryl sulphamoyl)imine

4.94 g (20mmole)of3-(3-chloro-2-methylphenyl)-1,2,3,4-oxatriazole-5-imine hydrochloride were dissolved in 70 ml of water and 4.2 g (50 mmole) of sodium hydrogencarbonate were subsequently added while being stirred. After terminated development of carbon dioxide 70 ml of dichloromethane were added, whereafter the precipitated product dissolved. 5.02 g (20 mmole) of (1S)-(+)-camphor-10-sulphonic acid chloride were added to the mixture under vigorous stirring, and the mixture was then stirred for 16 hours. The organic phase was separated and then washed with 1N hydrochloric acid and subsequently with water, whereafter it was concentrated into an oil, and left to stand in a small quantity of ethanol said oil slowly formed a precipitate, which was separated by filtration and then dried under vacuum.
Yield: 3.76 g = 43.0%
Melting point: 126-128°C
IR: 1740 cm⁻¹ (-CO-); 1630 cm⁻¹, 1315 cm⁻¹, 1150 cm⁻¹ (N⁻-SO₂-)

| Elemental analysis: C₁₈H₂₁ClN₄SO₄: | | | | |
|---|---|---|---|---|
| Calculated | C: 50.88% | H: 4.98% | N: 13.19% | S: 7.55% |
| Found | C: 50.80% | H: 4.96% | N: 13.14% | S: 7.69% |

### Example 5

### 3-(3-chloro-2-methylphenyl)-1,2,3,4-oxatriazole-5-(N-4-carbethoxy phenyl carbamoyl)imine

4.9 g (20 mmole) of 3-(3-chloro-2-methylphenyl)-1,2,3,4-oxatriazole-5-imine hydrochloride were dissolved in 50 ml of water, to which 2.0 g (24 mmole) of sodium hydrogencarbonate were subsequently added while being stirred. After terminated development of carbon dioxide 50 ml of dichlorometlane were added, whereafter the precipitated product dissolved. 3.82 g (20 mmole) of 4-isocyanatobenzoic acid ethyl ester were added to said mixture under vigorously stirring, whereby the end product precipitated almost instantaneously. The mixture was stirred for a further 30 minutes, whereafter the precipitated product was separated by filtration, washed thoroughly with water and subsequently with diethyl ether, and dried under vacuum.
Yield: 7.07 g = 88.0%
Melting point: 183-185°C
IR: 1680 cm⁻¹, 1270 cm⁻¹, 1110 cm⁻¹ (ester); 1635 cm⁻¹ (N-CO-NH).

| Elemental analysis: C₁₈H₁₆ClN₅O₄, 1/3 H₂O | | | |
|---|---|---|---|
| Calculated | C: 53.01% | H: 4.12% | N: 17.18% |
| Found | C: 53.03% | H: 4.04% | N: 16.83% |

### Example 6

### 3-(3-chloro-2-methylphenyl)-1,2,3,4-oxatriazole-5-(N-4-methoxy-phenylsulphonyl carbamoyl)imine

4.94 g (20 mmole) of 3-(3-chloro-2-methylphenyl)-1,2,3,4-oxatriazole-5-imine hydrochloride were dissolved in 70 ml of water, to which 2.5 g (30 mmole) of sodium hydrogencarbonate were subsequently added while being stirred. After terminated development of carbon dioxide 70 ml of dichloromethane were added, whereafter the precipitated product dissolved. 4.5 g (21 mmole) of 4-methoxy benzene sulphonyl isocyanate were added to the mixture under vigorous stirring, and the mixture was then stirred for 2 hours, whereafter the organic phase was separated. The organic phase was washed trice with 50 ml of 1N hydrochloric acid and subsequently with water, whereafter the mixture was concentrated. The residue was stirred thoroughly with 100 ml of diethyl ether, whereafter the product was separated by filtration and dried.
Yield: 6.34 g = 72.7%
Melting point: 135°C
IR: 1690 cm⁻¹, 1330 cm⁻¹, 1160 cm⁻¹ (N⁻-SO₂-); 1620 cm⁻¹ (N⁻-CO-NH); 1260 cm⁻¹ (OCH₃).

| Elemental analysis: C₁₆H₁₄ClN₅O₅S: | | | | |
|---|---|---|---|---|
| Calculated | C: 45.34% | H: 3.33% | N: 16.53% | S: 7.36% |
| Found | C: 45.05% | H: 3.34% | N: 16.38% | S: 7.23% |

### Example 7

### 3-(3-chloro-2-methylphenyl)-1,2,3,4-oxatriazole-5-(N-carbethoxy-carbamoyl)imine

2.47 g (10 mmole) of 3-(3-chloro-2-methylphenyl)-1,2, 3,4-oxatriazole-5-imine hydrochloride were dissolved in 30 ml of water and 0.9 g (10.7 mmole) of sodium hydrogen carbonate were subsequently added while being stirred. After the development of carbon dioxide was terminated, 30 ml of dichloromethane were added, whereafter the precipitated product dissolved. 1.15 g (10 mmole) of carbethoxy ethyl isocyanate were added to the mixture during vigorous stirring, whereafter the mixture was stirred for 30 minutes. The organic phase was separated and washed with water, whereafter the mixture was concentrated and the residue was washed with ether. The product was subsequently separated by filtration and dried.
Yield: 2.55 g = 78.3 %
Melting point: 123 to 127°C
IR: 1770 cm⁻¹, 1200 cm⁻¹ (ester); 1640 cm⁻¹ (N⁻-CO-NH).

| Elemental analysis: C₁₂H₁₂ClN₅O₄: | | | |
|---|---|---|---|
| Calculated | C: 44.25% | H: 3.71% | N: 21.50% |
| Found | C: 44.18% | H: 3.72% | N: 21.08% |

### Example 8

### 3-(3-chloro-2-methylphenyl)-1,2,3,4-oxatriazole-5-(N-carbethoxy methylcarbamoyl)imine

4.9 g (20 mmole) of 3-(3-chloro-2-methylphenyl)-1,2,3,4-oxatriazole-5-imine hydrochloride were dissolved in 60 ml of water and 1.8 g (21 mmole) of sodium hydrogencarbonate were subsequently added while being stirred. After terminated development of carbon dioxide 60 ml of dichloromethane were added, whereafter the precipitated product dissolved. 2.5 g (20 mmole) of ethoxy carbonyl methyl isocyanate were added to the mixture undet vigorous stirring, whereafter the mixture was stirred for 30 minutes, whereby a precipitate was formed. Dichloromethane was added causing the precipitate to dissolve. The organic phase was separated and subsequently washed with water and concentrated, wherafter the residue was stirred with a small quantity of ether, separated by filtration and dried.
Yield: 5.9 g = 86.8%
Melting point: 163-165°C
IR: 1755 cm⁻¹, 1200 cm⁻¹ (ester); 1645 cm⁻¹, 1635 cm⁻¹ (N⁻-CO-NH).

| Elemental analysis: C₁₃H₁₄ClN₅O₄: | | | |
|---|---|---|---|
| Calculated | C: 45.96% | H: 4.15% | N: 20.61% |
| Found | C: 46.10% | H: 4.22% | N: 20.36% |

### Example 9

### 3-(3-chloro-2-methylphenyl)-1,2,3,4-oxatriazole-5(N-2-carbamoyl-3-phenyl propionic acid ethyl ester)imine

4.9 g (20 mmole) of 3-(3-chloro-2-methyl)-1,2,3,4-oxatriazole-5-imine hydrochloride were dissolved in 60 ml of water and 1.8 g (24 mmole) of sodium hydrogencarbonate were subsequently added while being stirred. After terminated development of carbon dioxide 60 ml of dichloromethane were added, whereafter the precipitated product dissolved. 4.38 g (20 mmole) of 2-isocyanato-3-phenyl propionic acid ethyl ester were added to the mixture being stirred vigorously, whereupon the mixture was stirred for 30 minutes. The organic phase was separated and then washed with 1N hydrochloric acid and subsequently with water, whereafter it was concentrated. The residue was washed with a small quantity of diethyl ether, then separated by filtration and dried under vacuum.
Yield: 5.0 g = 58.2%
Melting point: 127-128°C
IR: 1745 cm⁻¹ (ester); 1670 cm⁻¹, 1630 cm⁻¹ (N⁻-CO-NH).

| Elemental analysis: C₂₀H₂₀ClN₅O₄: | | | |
|---|---|---|---|
| Calculated | C: 55.88% | H: 4.69% | N: 16.29% |
| Found | C: 55.79% | H: 4.53% | N: 16.04% |

### Example 10

### 3-(3-chloro-2-methylpheny1)-1,2,3,4-oxatriazole-5-(N-2-carbamoylpropionic acid methyl ester)imine

4.9 g (20 mmole) of 3-(3-chloro-2-methylphenyl)-1,2,3,4-oxatriazole-5-imine hydrochloride were dissolved in 60 ml of water and 1.8 g (24 mmole) of sodium hydrogencarbonate were subsequently added while being stirred. After terminated development of carbon dioxide 60 ml of dichloromethane were added, whereafter the precipitated product dissolved. 2.58 g (20 mmole) of 2-isocyanate propionic acid methyl ester were added to the mixture being stirred vigorously, whereupon the mixture was stirred for 30 minutes. The organic phase was separated and then washed with 1N hydrochloric acid and subsequently with water, whereafter it was concentrated. The residue was washed with a small quantity of diethyl ether, then separated by filtration and dried under vacuum.
Yield: 3.5 g = 51.5%
Melting point: 66-69°C
IR: 1745 cm⁻¹, 1200 cm⁻¹ (ester); 1670 cm⁻¹, 1635 cm⁻¹ (N⁻-CO-NH).

| Elemental analysis: C₁₃H₁₄ClN₅O₄: | | | |
|---|---|---|---|
| Calculated | C: 45.96% | H: 4.15% | N: 20.61% |
| Found | C: 45.54% | H: 4.14% | N: 20.11% |

### PHARMACOLOGiCAL TESTS

### 1. Inhibition of blood platelet aggregation

Compounds according to the invention were tested for their inhibition of clumping together (aggregation) of blood platelets (thrombocytes), which is the first phase of the formation of blood clots (thrombi). Such an inhibition may prevent the formation of blood clots and inhibit the development of new thrombi after a diagnosed thrombus.

The method of demonstrating this effect is a so-called aggregometer measurement, which was first described by Born (Nature (Lond.) 194, 927-929, 1962). Citrate stabilized ( 0.38% of sodium citrate, final concentration) venous blood from healthy testees is used, who have not used medicine for at least 8 days. Slight centrifugation (160 x g for 10 minutes) results in PRP (blood plasma rich in platelets) which is pipetted. PPP (blood plasma poor in platelets) is obtained by an intense centrifugation (3000 x g for 10 minutes) of the remaining blood. The light transmission is measured by the aggregometer (CHRONOLOG). PRP allows nearly no light transmission, while PPP allows complete transmission of light. The PRP is placed in the aggregometer at 37°C while being stirred by a magnet. Addition of a pro-aggregating substance causes the PRP to aggregate gradually and an increasing light transmission takes place at the same time. At complete aggregation a light transmission corresponding to PPP is obtained. Adenosine diphosphate (ADP) is used as pro-aggregating substance, said substance representing a basic biochemical mechanism for aggregation of blood platelets. The test substances are incubated for three minutes in PRP placed in the aggregometer at 37°C during magnetic stirring. A predetermined positively aggregating dosage of adenosine diphosphate (ADP) (2 to 4 µM) is then added. At least three different concentrations of the test substances are tested to demonstrate dosage-dependent inhibition of the aggregation. A so-called IC₅₀-value (that is the concentration inhibiting the aggregation by 50% relative to the control aggregation) is calculated for each test substance by linear regression analysis (log concentration µM as constant ad abscissa and % inhibition as variable ad ordinate). The following known reference substances have been used: nitroglycerine (GTN), sodium nitroprusside (NNP) and SIN-1 (the active metabolite of molsidomine).

The results for ten compounds according to the invention and three reference substances appear from Table 1.

It appears from Table 1 that the compounds according to the invention in general are substantially superior to the best of the reference substances. Nitroglycerine appears to be inactive in this test.

**Table 1**

| INHIBITION OF BLOOD PLATELET AGGREGATION | |
|---|---|
| Compound | IC₅₀, µM |
| GTN | 100 |
| NNP | 2.7 |
| SIN-1 | 3.9 |
| Compound prepared according to Example No. | |
| 1 | 0.49 |
| 2 | 0.37 |
| 3 | 1.01 |
| 4 | 0.08 |
| 5 | 2.98 |
| 6 | 0.67 |
| 7 | 0.16 |
| 8 | 7.60 |
| 9 | 7.90 |
| 10 | 2.31 |

### 2. Relaxation effect on the trachea

Compounds according to the invention were tested for their ability to relax a pre-contracted trachea. A contraction of the respiratory passages in combination with a swelling of the mucuos membrane therein presents a vital factor at asthmatic conditions. Relaxation or dilation of the contracted respiratory passages will improve the asthmatic condition.

The method of demonstrating relaxation of a pre-contracted trachea is described by Emmerson & MacKay (J. Pharm. Pharmacol 31, 798, 1979). An isolated trachea from a guinea pig is used. After preparation of a strip which has maintained the circular musculature, the organ strip is divided into two parts of equal size. The two tracheal strips are suspended in their respective organ bath and connected to a transducer recording the contraction and relaxation of the organ by means of a recorder. The two tracheal strips are continuously bathed in a Krebs buffer at 37°C, constantly bubbled with carbogen (95% O₂ and 5% CO₂). After an equilibration time of about 3 hours the organ strips are tested for their sensitivity (contractility) to carbamylcholin, a bolus being added directly to the bath (0.3 µM). If the contraction is satisfactory, the organ strips are transferred to the Krebs buffer containing the same concentration of carbamylcholine. The organ strips are now constantly exposed to the carbamylcholine and slowly develop a permanent contraction ("asthma"). The test compounds are added directly to the organ bath in bolus form. Having reached maximum effect (relaxation), the added substances are rinsed out of the system and the tracheal strip reverts to its permanent contraction state. At least three different concentrations of the test compounds are tested to demonstrate a dose-dependent relaxation of the organ. An EC₅₀-value (that is the concentration relaxing the organ by 50% relative to the maximum relaxation) is calculated for each test compound by means of a linear regression analysis (log concentration (µM) as a constant ad abscissa and % relaxation as variable ad ordinate). Sodium nitroprusside (NNP) and SIN-1 are used as reference compounds.

The results for ten compounds according to the invention and for the two reference compounds appear from Table 2.

It appears from Table 2 that the compounds according to the invention have a strong relaxing effect on the pre-contracted tracheal strips, said effect being in agreement with the effect of NNP and SIN-1. In addition, however, the compounds according to the invention have a longer lasting effect than the reference compounds.

**Table 2**

| RELAXATION OF THE TRACHEA | |
|---|---|
| Compound | IC₅₀ µM |
| NNP | 2.5 |
| SIN-1 | 18.3 |
| Compound prepared according to Example No. | |
| 1 | 3.9 |
| 2 | 13.9 |
| 3 | 11.1 |
| 4 | 0.90 |
| 5 | 34 |
| 6 | 3.9 |
| 7 | 3.0 |
| 8 | 11 |
| 9 | 37 |
| 10 | 23 |

The invention has been described with reference to preferred embodiments. Many modifications may, however, be carried out without thereby deviating from the scope of the invention.

## Claims

1. 3- and 5-substituted 1 ,2,3,4-oxatriazole-5-imine compounds of the general formula I 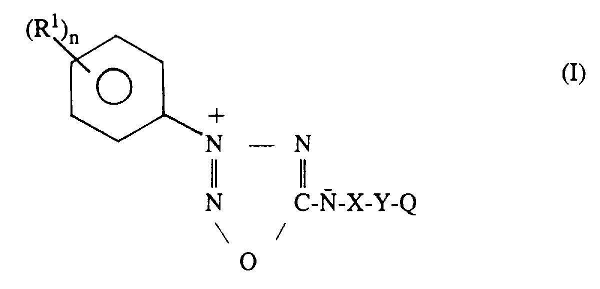 **characterised** in that
R¹ is the same or different groups and represents alkyl or alkoxy groups having 1 to 3 carbon atoms, halogen, trifluoromethyl, nitro, cyano, phenyl or alkylsulphonyl groups, n is 1 to 3, whereby R¹ is not halogen or alkyl, when n = 1,
X is -SO₂ or - C(O)NH-,
Y is -(CHR)ₘ-, wherein m = 1 to 4, and R means -CH₂-aryl, alkyl, hydrogen or a direct bond, and
Q means 10-camphoryl, -C(O)O-alkyl, aryl, -SO₂-alkyl or -SO₂-aryl, where aryl means phenyl or 4-alkyl-1,3-thiazole-5-yl and the aryl group is substituted by 1 to 3 groups Z, where Z means -NH-C(O)-C₁₋₆alkyl, -C(O)O-C₁₋₆alkyl or -O- (CHR³)ₚ-OH, wherein p = 1 to 4 and R³ means H or OH, and Z may further mean methoxy, when the aryl group in -SO₂-aryl is a phenyl group.

2. A compound as claimed in claim 1, **characterised** in that it is 3-(3-chloro-2-methylphenyl)-1,2,3,4-oxatriazole-5-(N-(1S)-(+)-10-camphorylsulphamoyl)imine.

3. A compound as claimed in claim 1, **characterised** in that it is 3-(3-chloro-2-methylphenyl)-1,2,3,4-oxatriazole-5-(N-2-acetamido-4-methyl-5-thiazole sulphamoyl)imine.

4. A compound as claimed in claim 1, **characterised** in that it is 3-(3-chloro-2-methylphenyl)-1,2,3,4-oxatriazole-5-(N-4-(methoxyphenyl sulphonyl carbamoyl)imine.

5. A pharmaceutical preparation, **characterised** in that it comprises a compound of the general formula I according to claim 1 as an active ingredient together with a pharmaceutically acceptable carrier or diluent.

6. A process for the preparation of 3- and 5-substituted 1,2,3,4 oxatriazole-5-imine compounds of the general formula I according to claim 1, **characterised** by ring closing a 1-arylthiosemicarbazide derivative of the general formula II 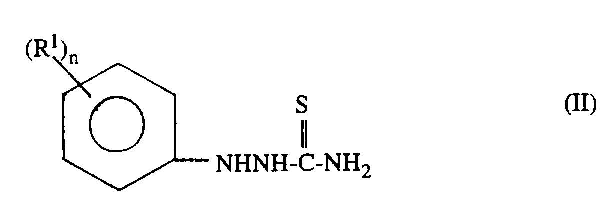 wherein R¹ and n have the same meaning as in formula I, by treatment with alkyl nitrite having 1 to 6 carbon atoms or alkali metal nitrite under acidic conditions at 0 to 10°C, whereafter the resulting salt is converted into the corresponding free compound, which is subsequently reacted with a compound of the type ClSO₂-Y-Q or O=C=N-Y-Q, wherein Y and Q have the same meaning as in formula I.

7. The use of 3- and 5-substituted 1,2,3,4,-oxatriazole-5-imine compounds of the general formula I according to claim 1 for the preparation of a medicament for the treatment of asthma.

8. The use of 3- and 5-substituted 1,2,3,4-oxatriazole-5-imine compounds of the general formula I according to claim 1 for the preparation of a medicament having an inhibiting effect on the blood platelet aggregation.

9. The use of 3- and 5-substituted 1,2,3,4-oxatriazole-5-imine compounds of the general formula I according to claim 1 for the preparation of a medicament being effective against impotence.

10. The use of 3- and 5-substituted 1,2,3,4-oxatriazole-5-imine compounds of the general formula I according to claim 1 for the preparation of a medicament being effective against pre-eclampsia.

## Patentansprüche

1. 3- und 5-substituierte 1,2,3,4-Oxatriazol-5-iminverbindungen der allgemeinen Formel I 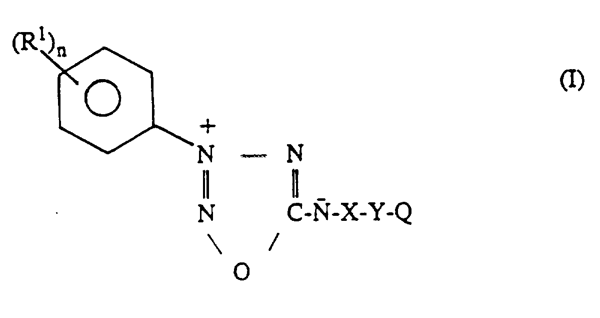 dadurch gekennzeichnet, daß
R¹ dieselbe Gruppe oder verschiedene Gruppen bedeutet und Alkyl- oder Alkoxygruppen mit 1 bis 3 Kohlenstoffatomen, Halogen-, Trifluormethyl-, Nitro-, Cyan-, Phenyl- oder Alkylsulfonylgruppen darstellt, n gleich 1 bis 3 ist, wobei R¹ nicht Halogen oder Alkyl ist, wenn n = 1,
X -SO₂ oder -C(O)NH- ist,
Y -(CHR)ₘ- ist, wobei m = 1 bis 4 und R -CH₂-Aryl, Alkyl, Wasserstoff oder eine direkte Bindung bedeutet und
Q 10-Camphoryl, -C(O)O-Alkyl, Aryl, -SO₂-Alkyl oder -SO₂-Aryl bedeutet, wobei Aryl Phenyl oder 4-Alkyl-1,3-thiazol-5-yl bedeutet und die Arylgruppe durch 1 bis 3 Gruppen Z substituiert ist, wobei Z -NH-C(O)-C₁₋₆ Alkyl, -C(O)O-C₁₋₆ Alkyl oder -O-(CHR³)ₚ-OH bedeutet, wobei p = 1 bis 4 und R³ H oder OH bedeutet, und Z ferner Methoxy bedeuten kann, wenn die Arylgruppe in -SO₂-Aryl eine Phenylgruppe ist.

2. Verbindung nach Anspruch 1, dadurch gekennzeichnet, daß sie 3-(3-Chloro-2-methylphenyl)-1,2,3,4-oxatriazol-5-(N-(1S)-(+)-10-camphorylsulfamoyl)-imin ist.

3. Verbindung nach Anspruch 1, dadurch gekennzeichnet, daß sie 3-(3-Chloro-2-methylphenyl)-1,2,3,4-oxatriazol-5-(N-2-acetamido-4-methyl-5-thiazolsulfamoyl)-imin ist.

4. Verbindung nach Anspruch 1, dadurch gekennzeichnet, daß sie 3-(-3-Chloro-2-methylphenyl)-1,2,3,4-oxatriazol-5-(N-4-(methoxyphenylsulfonylcarbamoyl)-imin ist.

5. Pharmazeutisches Präparat, dadurch gekennzeichnet; daß es eine Verbindung der allgemeinen Formel I gemäß Anspruch 1 als einen Wirkstoff zusammen mit einem pharmazeutisch annehmbaren Träger oder Verdünnungsmittel aufweist.

6. Verfahren zur Herstellung von 3- und 5-substituierten 1,2,3,4-Oxatriazol-5-iminverbindungen der allgemeinen Formel I gemäß Anspruch 1, gekennzeichnet durch Ringschluß eines 1-Arylthiosemicarbazidderivats der allgemeinen Formel II 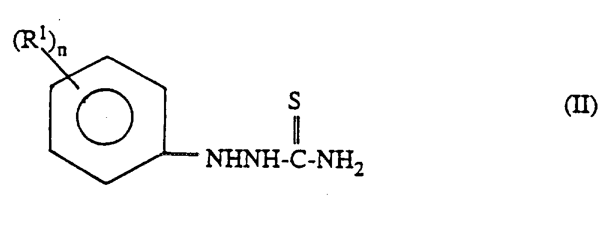 wobei R¹ und n dieselbe Bedeutung wie in der Formal I haben, durch Behandlung mit Alkylnitrit mit 1 bis 6 Kohlenstoffatomen oder Alkalimetallnitrit unter sauren Bedingungen bei 0 bis 10°C, wonach das resultierende Salz in die entsprechende freie Verbindung umgewandelt wird, die anschließend zur Reaktion gebracht wird mit einer Verbindung des Typs ClSO₂-Y-Q oder O=C=N-Y-Q, wobei Y und Q dieselbe Bedeutung wie in der Formel I haben.

7. Verwendung von 3- und 5-substituierten 1,2,3,4-Oxatriazol-5-iminverbindungen der allgemeinen Formel I gemäß Anspruch 1 zur Herstellung eines Medikaments für die Behandlung von Asthma.

8. Verwendung von 3- und 5-substituierten 1,2,3,4-Oxatriazol-5-iminverbindungen der allgemeinen Formel I gemäß Anspruch 1 zur Herstellung eines Medikaments mit einer inhibierenden Wirkung auf die Blutplättchenaggregation.

9. Verwendung von 3- und 5-substituierten 1,2,3,4-Oxatriazol-5-iminverbindungen der allgemeinen Formel I gemäß Anspruch 1 zur Herstellung eines Medikaments, das wirksam gegen Impotenz ist.

10. Verwendung von 3- und 5-substituierten 1,2,3,4-Oxatriazol-5-iminverbindungen der allgemeinen Formel I gemäß Anspruch 1 zur Herstellung eines Medikaments, das wirksam gegen Präeklampsie ist.

## Revendications

1. Composés 1,2,3,4-oxatriazole-5-imines substitués en positions 3 et 5 de formulé générale I 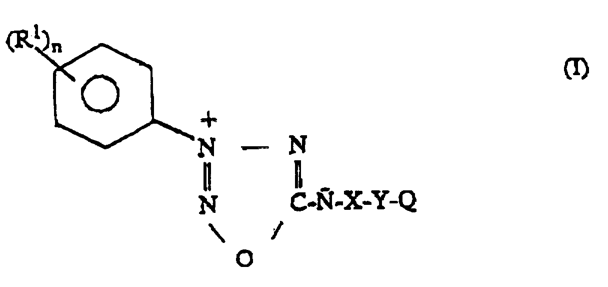 caractérisés en ce que
R¹ représente le même groupe ou des groupes différents et représente des groupes alkyles ou alcoxy ayant 1 à 3 atomes de carbone, des halogènes, des groupes trifluorométhyle, nitro, cyano, phényle où alkylsulfonyle, n vaut de 1 à 3, où R¹ n'est pas un halogène ni un groupe alkyle lorsque n = 1,
X est le groupe -SO₂ ou -C(O)NH-,
Y est un groupe (CHR)ₘ ⁻, dans lequel m = 1 à 4, et R
désigne un groupe -CH₂-aryle, alkyle, un atome d'hydrogène ou une liaison directe, et
Q désigne un groupe 10-camphoryle, -C-(O)O-alkyle, aryle, -SO₂-alkyle ou -SO₂-aryle, où aryle désigne un groupe phényles ou un groupe 4-alkyl-1,3-thiazol-5-yle et le groupe aryle est substitué par 1 à 3 groupes 2, où Z désigne un groupe -NH-C(O)-(alkyle en C₁₋₆), -C(O)O-(alkyle en C₁₋₆) ou -O-(CHR³)ₚ-OH, où p = 1 à 4 et R³ désigne H ou OH, et Z peut de plus désigner un groupe méthoxy lorsque le groupe aryle dans -SO₂-aryle est un groupe phényle.

2. Composé selon la revendication 1, caractérisé en ce qu'il s'agit de la 3-(3-chloro-2-méthylphényl)-1,2,3,4-oxatriazole-5-(N-(1S)-(+)-10-camphorylsulfamoyl)imine.

3. Composé selon la revendication 1, caractérisé, en ce qu'il s'agit de la 3-(3-chloro-2-méthylphényl)-1,1,3,4-oxatriazole-5-(N-2-acétamido-4-méthyl-5-thiazolesulfamoyl)imine.

4. Composé selon la revendication 1, caractérisé en ce qu'il s'agit de la 3-(3-chloro-2-méthylphényl)-1,2,3,4-oxatriazole-5-(N-4-(méthoxyphénylsulfonylcarbamoyl)imine.

5. Préparation pharmaceutique, caractérisée en ce qu'elle comprend un composé de formule générale I selon la revendication 1 comme principe actif, ainsi qu'un véhicule du revendication 1 comme principe actif, ainsi qu'un véhicule ou un diluant acceptable d'un point de vue pharmaceutique.

6. Procédé de préparation des composés 1,2,3,4-oxatriazole-5-imines substitués en positions 3 et 5 de formule générale I selon la revendication 1, caractérisé par la cyclisation d'un dérivé 1-arylthiosemicarbazide de formule générale II 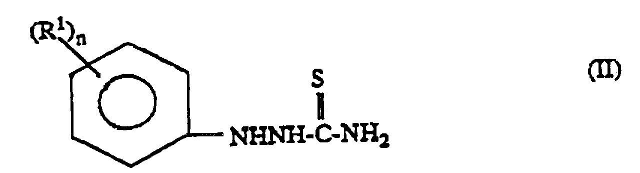 dans laquelle R¹ et n ont les mêmes significations que dans la formule I, par le traitement avec un nitrite d'alkyle ayant de 1 à 6 atomes de carbone ou un nitrite de métal alcalin, dans des conditions acides, entre 0 et 10°C, ensuite le sel résultant est converti en composé libre correspondant, est mis à réagir, par la suite, avec un composé du type ClSO₂-Y-Q ou O=C=N-Y-Q, où Y et Q ont les mêmes significations dans la formule I.

7. Utilisation des composés 1,2,3,4-oxatriazyle-5-imines substitués en positions 3 et 5 de formule générale I selon la revendication 1, pour la préparation d'un médicament destiné au traitement de l'asthme.

8. Utilisation des composés 1,2,3,4-oxatriazle-5-imines substitués en positions 3 et 5 de formule générale I selon la revendication 1, pour la préparation d'un médicament ayant un effets inhibiteur sur l'agrégation des plaquettes sanguines.

9. Utilisation des composés 1,2,3,4-oxatriazole-5-imines substitués en positions 3 et 5 de formule générale I selon la revendication 1, pour la préparation d'un médicament qui est efficace contre l'impuissance.

10. Utilisation des composés 1,2,3,4-oxatriazole-5-imines substitués en positions 3 et 5 de formule générale I selon la revendication 1, pour la préparation d'un médicament qui est efficace contre la pré-éclampsie.
